# FASCICULE DE BREVET EUROPEEN

(11) **EP 1 744 775 B1**
(45) Date de publication et mention de la délivrance du brevet: **03.08.2016**
(21) Numéro de dépôt: 05767512.6
(22) Date de dépôt: 29.04.2005
(51) Int. Cl.: A61K 31/198, A61K 36/54, A61P 17/02, A61Q 19/00

(54) **MEDICAMENT COMPRENANT UN EXTRAIT PEPTIDIQUE D'AVOCAT DESTINE AU TRAITEMENT ET A LA PREVENTION DES MALADIES LIEES A UNE DEFICIENCE DU SYSTEME IMMUNITAIRE**
MEDIKAMENT MIT AVOCADO-PEPTIDEXTRAKT ZUR BEHANDLUNG UND VORBEUGUNG VON ERKRANKUNGEN IN ZUSAMMENHANG MIT EINER IMMUNSYSTEMFEHLFUNKTION
MEDICAMENT COMPRISING A PEPTIDE EXTRACT OF AVOCADO, WHICH IS INTENDED FOR THE TREATMENT AND PREVENTION OF ILLNESSES THAT ARE LINKED TO AN IMMUNE SYSTEM DEFICIENCY

(30) Priorité: 30.04.2004 FR 0404640
(43) Date de publication de la demande: 24.01.2007
(73) Titulaire: Laboratoires Expanscience, 92400 Courbevoie (FR)
(72) Inventeur: PICCIRILLI, Antoine, F-78670 Villennes sur Seine (FR); PICCARDI, Nathalie, F-21310 Arceau (FR); MSIKA, Philippe, F-78000 Versailles (FR); PAUL, François, 09160 Montgauch (FR); BREDIF, Stéphanie, F-28210 Chaudon (FR)
(74) Mandataire: Regimbeau
(86) Numéro de dépôt international: PCT/FR2005/001076
(87) Numéro de publication internationale: WO 2005/105123

(56) Documents cités:
- WO-A-01/68040
- WO-A-99/43298
- DE-A- 19 852 508
- FR-A- 2 787 714
- KASHMAN Y ET AL: "New compounds from avocado pear" 1969, TETRAHEDRON, ELSEVIER SCIENCE PUBLISHERS, AMSTERDAM, NL, PAGE(S) 4617-4631 , XP002090295 ISSN: 0040-4020 le document en entier

## Description

La présente invention concerne un médicament comprenant un extrait peptidique d'avocat, avantageusement destiné au traitement et/ou à la prévention de maladies liées à une déficience du système immunitaire, et plus particulièrement à une altération de l'immunité innée.

Toutes les espèces animales sont confrontées de façon quotidienne à un grand nombre de microorganismes, tels que des bactéries, des champignons, des parasites ou des virus, qui peuvent affecter leur santé voire leur survie. Deux systèmes de défense s'opposent à ces microorganismes : un système appelé immunité innée, qui est commun à tous les animaux, y compris l'homme, et un système immunitaire dit adaptatif ou spécifique qui est acquis grâce aux cellules et aux médiateurs de l'immunité après contact avec l'agresseur potentiel.

Une différence entre les réponses immunitaires innées ou adaptatives se situe au niveau des mécanismes de reconnaissance des microorganismes impliqués. Dans l'immunité innée, la spécificité des récepteurs est génétiquement déterminée dès la naissance et elle n'est pas variable. Ces récepteurs sont exprimés sur des cellules telles que certaines cellules épithéliales et endothéliales, les cellules dendritiques, les monocytes et les macrophages. Toutes les structures reconnues par les récepteurs de l'immunité innée sont communes à de très nombreux microorganismes. Contrairement à la réponse immunitaire adaptative, les mécanismes de la réponse immunitaire innée (phagocytose, peptides antimicrobiens, ...) sont activés dès le début de l'infection et contrôlent de façon quasi-immédiate la prolifération des pathogènes qui envahissent l'hôte. Ensuite, la réponse immunitaire adaptative prend le relais.

Les peptides anti-microbiens ont été retrouvés à la fois dans le règne végétal et animal, et plus de 500 peptides anti-microbiens différents ont été découverts des insectes à l'homme. Les peptides anti-microbiens sont des molécules de petite taille (10 à 50 acides aminés) capables de détruire une grande variété de micro-organismes (bactéries Gram+ et/ou Gram-, champignons, virus, cellules transformées), en perméabilisant leur membrane cellulaire. En outre, certains de ces peptides anti-microbiens via des propriétés chemo-attractives sont capables de recruter des cellules participant à l'immunité adaptative telles que les cellules dendritiques ou bien encore les lymphocytes T. De nombreux peptides anti-microbiens ont été mis en évidence dans le vernix caseosa et dans le fluide amniotique, ainsi que dans la peau des nouveaux nés, suggérant leur rôle clé dans la défense anti-microbienne lors de l'accouchement, mais également dès les début de la vie alors que l'immunité acquise est encore immature.

La plupart des organismes synthétisent plusieurs types de peptides anti-microbiens au niveau de leurs différents épithélia, de manière à définir un large spectre d'activité. Chez les mammifères, deux grandes classes de peptides anti-microbiens, dont la production est induite lors d'un contact avec un micro-organisme, ont été décrites : les cathélicidines et les défensines.

La cathélicidine humaine (LL-37) a été isolée pour la première fois à partir de cellules de la moelle osseuse. LL-37 est exprimée notamment dans la peau humaine, au niveau des ongles, ainsi qu'au niveau de la membrane synoviale saine et inflammatoire, notamment chez des patients atteints d'arthrose. LL-37 possède un large spectre d'activité, et semble agir en synergie avec d'autres peptides antimicrobiens notamment les défensines. LL-37 possède également des propriétés chémo-attractantes, ce qui lui confère la capacité de pouvoir recruter des cellules de l'immunité adaptative.

Les défensines sont elles-mêmes divisées en deux familles, α et β, sur la base de leur structure secondaire. Les α-défensines (6 connues à ce jour) sont principalement localisées dans les granules de stockage des cellules spécialisées telles que les neutrophiles, ou les cellules de Paneth intestinales, alors que les β-défensines sont caractéristiques des tissus épithéliaux. Outre leur rôle dans l'immunité innée, les défensines sont également connues pour leurs propriétés mitogéniques, ce qui suggère leur implication potentielle dans les processus de cicatrisation.

Chez l'homme, 4 β-défensines ont été identifiées à ce jour (il existerait plus de 20 gènes codant pour des peptides anti-microbiens dans notre génome). La β-défensine 1 (hBD-1) humaine est généralement produite de manière constitutive et est exprimée en quantité importante dans les reins et dans une moindre proportion au niveau du pancréas, des glandes salivaires, des épithélia des voies respiratoires, dans le système uro-génital de la femme, dans la membrane synoviale saine, ainsi que dans le placenta. hBD-1 est également exprimée dans la peau. Les autres formes de β-défensines, hBD-2, 3 et 4, sont inductibles. hBD-3 est induite au niveau des membranes synoviales inflammatoires comme par exemple dans les pathologies arthrosiques. L'expression de hBD-2 a été rapportée, à ce jour, dans la peau, le tractus uro-génital, les glandes sudorales, et au niveau de l'unité pilo-sébacée.

Au niveau de la peau, d'autres peptides ou protéines, tels que l'adrénomedulline, la cystatine, l'inhibiteur spécifique de l'élastase/SKALP/elafin, posséderaient des activités anti-microbiennes. Plus récemment, la dermicidine (large spectre d'activité) a été caractérisée comme un peptide anti-microbien spécifique de la peau qui serait produit au niveau des glandes sudorales eccrines, et dont la sécrétion simultanée avec la sueur constituerait une part importante du système de défense inné contre les infections locales et systémiques. hBD-2 a été caractérisée pour la première fois dans les squames de psoriasis. L'expression de hBD-2 et -3, ainsi que de LL-37, est augmentée au niveau des lésions de psoriasis, ce qui expliquerait la plus grande résistance aux infections des patients atteints de cette pathologie. A l'inverse, dans la dermatite atopique (lésions chroniques et lésions en poussée), l'expression de LL-37 et de hBD-2 est diminuée sous l'influence de l'interleukine-4 (IL-4) et de l'interleukine-13 (IL-13), médiateurs de l'atopie. Cette déficience pourrait expliquer la susceptibilité accrue aux infections des patients atteints de dermatite atopique. Dans l'acné, l'expression des β-défensines (hBD-1 et -2) est augmentée en réaction à la prolifération de *P acnes.* En outre, il est supposé que les acnéiques souffriraient d'un déséquilibre initial en peptides anti-microbiens, responsable de la prolifération bactérienne, bactéries qui en retour stimuleraient les défenses immunitaires innées.

L'inflammation semble donc être un facteur prépondérant dans l'induction des peptides anti-microbiens. Ainsi, il a été également monté que l'interleukine-1, le TNF-α (Tumor Necrosis alpha), et l'irradiation ultra-violette stimulaient la production de hBD-2. L'expression de hBD-2 est également liée à l'état de différenciation des kératinocytes. Ainsi, la stimulation de la production des peptides anti-microbiens, notamment de la famille des défensines, et plus particulièrement de hBD-2, permettrait de promouvoir et/ou de rétablir l'immunité innée, notamment au niveau des yeux et des épithélia (épiderme, muqueuses vaginale, intestinale, nasale et auriculaires, voies respiratoires).

La cavité buccale est constamment exposée à une grande variété de microbes (bactéries, virus, champignons). Entre autre, il est bien établi que des bactéries telles que actinobacillus actinomycetemcomitans, porphyromonas gingivalis sont des facteurs clé participant au développement des maladies parodontales (gingivite et parodontite). L'épithélium gingival constitue le premier rempart contre les différents pathogènes présents dans la sphère buccale. A ce titre, les kératinocytes gingivaux produisent un large panel de peptides anti-microbiens, hBD-1, -2, -3, LL37. Ces peptides sont également produits au niveau de la muqueuse buccale, ainsi que par les glandes salivaires.

Plus particulièrement, la stimulation des peptides antimicrobiens permettrait de promouvoir et/ou de rétablir l'immunité innée au niveau de la peau saine ou pathologique des nourrissons et des enfants, dont l'immunité est généralement déficiente, et au niveau de la peau des adultes ou des personnes âgées en bonne santé ou malades (immuno-déprimés). Cette stimulation permettrait ainsi de compléter avantageusement le système de défense passif de la peau que constitue le stratum corneum (cornéocytes + ciment intercellulaire), et de préparer la réponse immunitaire adaptative chez les nourrissons, les enfants, les adultes et les personnes âgées, en bonne santé ou malades. Parallèlement, cette stimulation permettrait de promouvoir la cicatrisation.

D'une manière surprenante, les inventeurs ont découvert qu'une composition comprenant un extrait peptidique d'avocat permet l'augmentation de la production de peptides antimicrobiens, avantageusement de la hBD-2.

Ainsi, la présente invention a pour objet un médicament comprenant un extrait peptidique d'avocat, qui comprend 2 à 10 % en poids d'azote alpha-aminé, par rapport au poids de la matière sèche de l'extrait peptidique, et un excipient approprié.

Dans le cadre de la présente invention, on entend par les termes « azote alpha-aminé », la teneur en azote des peptides sous la forme de groupes alpha-aminés libres. La mesure de la teneur en azote alpha-aminé des peptides permet d'évaluer le degré d'hydrolyse des protéines ainsi que la masse molaire moyenne des peptides.

L'extrait peptidique d'avocat peut directement être obtenu à partir de n'importe quelle partie de l'avocat ou de l'avocatier, telle que le fruit, la peau, le noyau, la feuille ou les racines de l'avocatier. Il est aussi possible d'obtenir un extrait peptidique d'avocat à partir des co-produits de l'industrie de transformation de l'avocat, parmi lesquels on peut citer de façon non exhaustive : la pulpe fraîche d'avocat, la pulpe congelée, déshydratée, les tourteaux d'avocat issus des procédés d'extraction d'huile (extraction mécanique et/ou par solvant du fruit préalablement déshydraté), les matières solides déshuilées issues des procédés d'extraction d'huile par voie humide (procédé dit de centrifugation), les matières solides déshuilées issues des procédés d'extraction d'huile d'avocat par voie enzymatique, les purées d'avocat brutes (guacamole), les déchets solides issus des unités de production de ces purées. L'extrait est avantageusement obtenu à partir du fruit frais de l'avocatier. Les fruits pourront être choisis parmi les variétés *Hass, Fuerte, Ettinger, Bacon, Nabal, Anaheim, Lula, Reed, Zutano*, *Queen, Criola Selva, Mexicana Canta, Region Dschang, Hall, Booth, Peterson, Collinson Red,* plus avantageusement, les variétés *Hass, Fuerte et Reed.* De préférence, on retiendra les variétés *Hass, Fuerte*, *Ettinger et Bacon,* et plus avantageusement les variétés *Hass* et *Fuerte.*

Le fruit de l'avocatier est principalement constitué d'eau, de pulpe, d'huile et de noyau. Les proportions de ces constituants sont, à l'instar de toutes matières naturelles et végétales, extrêmement variables. Toutefois, on admet généralement les données de composition moyennes suivantes, exprimées en pourcentage de fruit frais, données dans le tableau 1 suivant :

**Tableau 1**

| | |
|---|---|
| Eau | 70-85 % |
| Protéines | 1,5-4,5 % |
| Lipides | 12-23 % |
| Sucres | 1,5-5 % |
| Fibres | 1,1-1,6 % |

Par rapport à la pulpe, les protéines de l'avocat représentent 1,5 à 2,5 % (J.P. Gaillard, l'Avocatier, Ed. G.P. Maisonneuve et Larose, 1987, pp 266-67). La répartition en acides aminés, exprimée en pourcentage en poids par rapport au poids total des acides aminés, est donnée dans le tableau 2 suivant :

**Tableau 2**

| | |
|---|---|
| Alanine | 5-7 |
| Arginine | 3-5 |
| Acide aspartique | 8-12 |
| Cystine-cysteine | < 1 |
| Acide glutamique | 11-13 |
| Glycine | 4-6 |
| Histidine | 4-6 |
| Isoleucine | 4-7 |
| Leucine | 8-11 |
| Lysine | 4-7 |
| Méthionine | 1-3 |
| Phénylalanine | 4-6 |
| Proline | 4-7 |
| Sérine | 4-6 |
| Thréonine | 4-6 |
| Tyrosine | 3-6 |
| Valine | 4-7 |

Les principaux acides aminés sont l'acide glutamique, l'acide aspartique et la leucine.

Par comparaison aux plantes oléoprotéagineuses classiques telles que le soja, le tournesol ou le colza, l'avocat est nettement plus pauvre en protéines. Par ailleurs, la relative richesse du fruit en fibres, rend très difficile l'accessibilité à ces protéines par les voies classiques, chimique ou biochimique. De plus, le caractère très peu hydrosoluble de ces macromolécules naturelles invite à préparer des fractions hydrolysées de ces protéines (peptides), présentant une très grande solubilité dans l'eau, et une bien meilleure biodisponibilité. Par ce biais, leur potentiel allergisant peut aussi être éliminé. L'invention aura donc également pour but, la préparation d'un extrait peptidique d'avocat, par une voie de synthèse ménagée et non dénaturante des protéines hydrolysées.

Plus particulièrement, l'extrait peptidique d'avocat est susceptible d'être obtenu par un procédé comprenant les étapes suivantes :
- obtention d'un tourteau d'avocat, avantageusement du fruit de l'avocat, par séchage puis extraction de l'huile (lipides); ensuite
- cryobroyage et délipidation complète dudit tourteau puis décantation, centrifugation et récupération du gâteau ; puis
- première hydrolyse en présence de glucanases, suivie d'une centrifugation et de l'élimination de la fraction soluble ;
- seconde hydrolyse en présence d'une ou plusieurs protéases, suivie d'une centrifugation et de l'élimination du culot ; ensuite
- concentration de la phase peptidique par nanofiltration ;
- décoloration, en présence de charbon actif par exemple, suivie d'une filtration simple (10 µm) puis d'une ultrafiltration (seuil de coupure de 10 kD) ; enfin
- le cas échéant, micro filtration stérilisante finale (0,2 µm), ajout de conservateur et conditionnement.

Selon une variante avantageuse de l'invention, la première étape du procédé consiste à sécher le fruit puis à le déshuiler. Ainsi, après tranchage du fruit en fines lamelles, son séchage peut être réalisé par l'ensemble des techniques connues de l'homme de métier, parmi lesquelles on peut citer le séchage à l'air chaud, la lyophilisation ou encore le séchage osmotique. D'une façon générale, la température lors de cette étape de séchage sera avantageusement maintenue, quelle que soit la technique employée, inférieure ou égale à 80°C. Dans le cadre du présent procédé, pour des raisons de facilité de mise en oeuvre et pour des raisons de coût, le séchage en séchoirs ventilés, en couche mince et sous courant d'air chaud, à une température comprise entre 70 et 75°C est préféré. La durée de l'opération peut varier de 5 à 72 heures.

Les lipides du fruit séché sont par la suite extraits soit par voie mécanique dans une presse à vis continue ou encore par voie chimique, à l'aide d'un solvant tel que l'hexane dans un extracteur de type Soxhlet ou dans un extracteur continu à bande, de type De Smet®, notamment selon le procédé décrit dans la demande FR 2 843 027, ou encore par un procédé utilisant le CO₂ supercritique. Parmi les intérêts majeurs du procédé, l'huile co-produite constitue un produit bien évidemment directement valorisable. C'est pourquoi, on préfère l'extraction des lipides par voie mécanique.

Le fruit sec et déshuilé, ou encore appelé tourteau peut subir ensuite les étapes suivantes :
- cryobroyage
- délipidation complet, notamment par un solvant alimentaire non toxique tel que l'éthanol et/ou l'acétone
- décantation, puis lavage du tourteau à l'eau,
- centrifugation, et récupération du gâteau,
- première hydrolyse en présence d'une ou plusieurs glucanases,
- centrifugation et élimination de la fraction soluble,
- seconde hydrolyse en présence d'une ou plusieurs protéases,
- centrifugation et élimination du culot,
- concentration par nanofiltration,
- décoloration en présence de charbon actif,
- filtration simple (10 µm) puis ultrafiltration (seuil de coupure de 10 kD),
- ajout de conservateur, microfiltration stérilisante finale (0,2 µm) et conditionnement.

L'extrait aqueux final peut contenir en poids 1 à 60 % de matière sèche, ou encore 3 à 20 % de matière sèche, de préférence 5 à 6 % de matière sèche. Par rapport au poids de la matière sèche, la teneur massique en azote alpha-aminé pourra être comprise entre 2 et 10 %, de préférence entre 5 et 7 %. Le pH d'une solution aqueuse à 1,2 % en poids d'extrait sec, sera généralement compris entre 3 et 6, plus avantageusement entre 4 et 5. Les données analytiques moyennes d'une solution aqueuse à 1,2 % en poids d'extrait sec, obtenue par le procédé décrit précédemment, sont données dans le tableau 3 suivant :

**Tableau 3**

| | | |
|---|---|---|
| Azote α-aminé (méthode dite « o-phthalaldéhyde » ou « ninhydrine ») (en pourcentage massique dans la matière sèche) | | 4-10 |
| Protéines (en pourcentage massique dans la matière sèche) (N x 6,25)¹ | | 10-30 |
| pH (dilution ¼) | | 4,5-7,0 |
| Absorbance (dilution ¼) | 420 nm | 0,1-0,6 |
| | 550 nm | 0,02-0,1 |

¹N x 6,25 correspond au dosage de l'azote total (N) d'un échantillon multiplié par un coefficient spécifique pour la protéine dosée. Quand on ne connaît pas précisément le coefficient des protéines dosées, on utilise par convention le coefficient 6,25.

Le tableau 4 suivant donne la composition moyenne en acides aminés de l'extrait peptidique obtenu par le procédé selon l'invention, dans lequel les valeurs sont exprimées en pourcentage en poids par rapport au poids total des acides aminés dosés. Les valeurs pour l'acide aspartique et l'acide glutamique incluent également les teneurs en asparagine et en glutamine, respectivement.

**Tableau 4**

| Acide aminé | Valeur minimale | Valeur maximale |
|---|---|---|
| Alanine | 6,4 | 7,8 |
| Arginine | 4,7 | 5,7 |
| Acide aspartique | 10,3 | 12,7 |
| Cystine-cystéine | 2,9 | 3,5 |
| Acide glutamique | 13,0 | 15,8 |
| Glycine | 5,3 | 6,5 |
| Histidine | 2,2 | 2,6 |
| Isoleucine | 4,8 | 5,8 |
| Leucine | 7,6 | 9,4 |
| Lysine | 3,0 | 3,8 |
| Méthionine | 1,2 | 1,6 |
| Phénylalanine | 4,7 | 5,7 |
| Proline | 4,1 | 5,2 |
| Sérine | 5,5 | 6,7 |
| Thréonine | 4,6 | 5,6 |
| Tyrosine | 3,6 | 4,4 |
| Valine | 5,8 | 7,2 |

| | | |
|---|---|---|
| Tryptophane non dosé | | |

L'extrait obtenu pourra éventuellement être lyophilisé dans le but d'obtenir une poudre solide (extrait sec), mais totalement hydrosoluble par rapport aux protéines originelles de l'avocat.

Selon une variante avantageuse de l'invention, 50 % au moins des peptides de l'extrait sont constitués de 10 à 30 motifs d'acides aminés. La taille de ces peptides est donc beaucoup plus petite par comparaison à celle des protéines natives de l'avocat. Ces peptides présentent donc à ce titre une bien meilleure bio disponibilité, notamment cutanée.

Le médicament selon l'invention est particulièrement approprié au traitement et/ou à la prévention de maladies liées à une modification de l'immunité innée et/ou acquise, par augmentation de la production de peptides antimicrobiens, de la famille des cathélicidines et/ou des bétâ-défensines, avantageusement de la hBD-2. Au sens de la présente invention, le terme « modification » peut signifier augmentation ou diminution.

Le médicament selon l'invention est également particulièrement approprié au traitement et/ou à la prévention de maladies liées à une modification de l'immunité innée et/ou acquise par stimulation des peptides anti-microbiens like, tels qu'un inhibiteur spécifique de l'élastase, particulièrement l'élafin (SKALP).

Le médicament selon l'invention permet avantageusement de stimuler et/ou de compléter l'immunité innée et/ou l'immunité acquise.

D'une manière générale, dans le cadre de la présente invention, lesdites maladies peuvent être liées à la présence de micro-organismes, notamment de bactéries Gram+ et/ou Gram-, de champignons ou de virus.

Plus particulièrement, lesdites maladies peuvent être des infections des systèmes oculaire et auditif, des épithélia non kératinisés (muqueuse vaginale, intestinale, gingivale, nasale, pulmonaire, tractus respiratoire, anale et uréthrale, pulmonaire) et kératinisés telle que la peau. Les dites maladies peuvent également être des infections des phanères ou annexes cutanées (cheveux, ongles, glandes sudorales, glandes sébaccées). Ainsi lesdites maladies peuvent être des pathologies telles que la folliculite, le furoncle, l'abcès, l'impétigo ou le panaris.

Lesdites maladies peuvent-être des pathologies du cuir chevelu telles que les pellicules, et plus largement des affections liées à une hyper-séborrhée.

Lesdites maladies peuvent également être des dermatoses inflammatoires, telles que la dermatite atopique, l'eczéma atopique et/ou de contact, le psoriasis, l'acné, et des dermatites irritatives.

Lesdites maladies peuvent aussi être des brûlures, en particulier des brûlures du premier ou second degré.

Lesdites maladies peuvent également être des pathologies liées à un déficit de la barrière cutanée. Ainsi, le médicament selon l'invention peut être utilisé pour le traitement des peaux hyper-réactives (sensibles, irritées, allergiques), atopiques, sèches ou âgées. Lesdites maladies peuvent aussi être des pathologies liées à des peaux fragilisées par une agression environnementale, notamment due au froid, à la pollution, au stress, au tabac, à des expositions solaires.

Dans le cadre de la présente invention, le médicament est également approprié pour la protection des peaux immatures, pathologiques, des nourrissons et des enfants. En effet, il permet de renforcer les défenses naturelles de l'épiderme de l'enfant dont l'immunité est généralement déficiente.

Dans le cadre de la présente invention, le médicament est également approprié pour la protection des peaux pathologiques des adultes ou des personnes âgées, notamment des individus immuno-déprimés.

Le médicament selon l'invention est également approprié pour favoriser la cicatrisation, dans les processus de cicatrisation normaux ou pathologiques, tels que les ulcères et les escarres.

Dans le cadre de la présente invention, le médicament est aussi destiné à traiter et/ou prévenir les maladies parodontales, les pathologies articulaires inflammatoires telle que l'arthrose, les infections des muqueuses, notamment des muqueuses vaginale, intestinale, respiratoire, nasale ou auriculaire, ou les infections du système oculaire.

Selon une variante avantageuse de l'invention, le médicament comprend 0,01 à 20% en poids sec d'extrait peptidique d'avocat, par rapport au poids total dudit médicament, encore plus avantageusement 0,1 à 15% en poids sec d'extrait peptidique d'avocat, encore plus avantageusement 0,5 à 10% en poids sec d'extrait peptidique d'avocat, encore plus avantageusement 0,7 à 8% en poids sec d'extrait peptidique d'avocat, et encore plus avantageusement 1 à 5% en poids sec d'extrait peptidique d'avocat.

Selon une variante avantageuse de l'invention, le médicament comprend en outre du D-mannoheptulose et/ou du perséitol (sucres en C7) ou d'un de leurs dérivés chimiques, avantageusement en une quantité de 0,001 à 30 % en poids sec, par rapport au poids total du médicament, encore plus avantageusement 0,01-20% en poids sec, encore plus avantageusement 0,1-10% en poids sec, encore plus avantageusement 0,5-5% en poids sec.

On observe alors avantageusement un effet de synergie.

La source de D-mannoheptulose et/ou de perséitol est avantageusement soit un extrait hydrosoluble de sucres d'avocat ou d'une autre plante. Autrement, le D-mannoheptulose et le perséitol sont disponibles commercialement (origine synthétique). Selon une variante avantageuse de l'invention, la source de D-mannoheptulose et/ou de perséitol est un extrait hydrosoluble de sucres d'avocat comprenant au moins 50% en poids, par rapport au poids total de la matière sèche de l'extrait, de sucres en C7.

L'extrait hydrosoluble de sucres d'avocat est susceptible d'être obtenu par un procédé comprenant les étapes successives suivantes :
- obtention d'un tourteau d'avocat, avantageusement du fruit de l'avocat, par séchage de l'avocat puis extraction des lipides (huile) ; ensuite
- cryobroyage et délipidation complète dudit tourteau, puis décantation et centrifugation afin de récupérer une fraction soluble riche en sucres en C7 (élimination du gâteau) ;
- déminéralisation sur résine ionique de ladite fraction soluble, obtenue à l'étape précédente ; puis
- ultrafiltration à 10 000 daltons ;
- le cas échéant, concentration par évaporation sous vide, ajout de conservateur, stérilisation par micro filtration (0,2 µm) et conditionnement.

Selon une variante avantageuse de l'invention, l'obtention du tourteau d'avocat et l'extraction des lipides sont avantageusement effectuées d'une manière identique pour l'extrait peptidique d'avocat et les sucres d'avocat.

Le fruit sec et déshuilé, ou encore appelé tourteau peut subir ensuite les étapes suivantes :
- cryobroyage
- délipidation complète, avantageusement à l'éthanol et/ou à l'acétone,
- décantation puis lavage du tourteau à l'eau,
- centrifugation et récupération de la fraction soluble (élimination du gâteau),
- déminéralisation par passage sur des résines échangeuses d'ions
- ultrafiltration avec un seuil de coupure de 10 kD,
- concentration sous vide, ajout de conservateur et conditionnement.

De façon générale, l'extrait aqueux final peut contenir 0,1 à 10 % en poids de matière sèche, avantageusement 1 à 7 % en poids de matière sèche, encore plus avantageusement 3 à 5 % en poids de matière sèche. La teneur en sucres en C7, c'est-à-dire en D-mannoheptulose et en perséitol, dans la matière sèche est avantageusement supérieure à 50% en poids, encore plus avantageusement comprise en 65 à 90% en poids, par rapport au poids total de la matière sèche.

La composition relative en sucres de l'extrait hydrosoluble d'avocat, en poids par rapport au poids total de la matière sèche de l'extrait, répond avantageusement aux critères suivants (composition relative déterminée par HPLC):

| | |
|---|---|
| - D-mannoheptulose | 5 à 80 % |
| - Perséitol | 5 à 80 % |
| - Saccharose | inférieur à 10% |
| - Glucose | inférieur à 10% |
| - Fructose | inférieur à 10% |

L'extrait hydrosoluble de sucre d'avocat comprend avantageusement, par rapport au poids total de la matière sèche, 1 à 99% en poids de mannoheptulose, plus avantageusement 5 à 80% en poids de mannoheptulose, encore plus avantageusement 10 à 80% en poids de mannoheptulose. L'extrait hydrosoluble de sucre d'avocat comprend avantageusement, par rapport au poids total de la matière sèche, 20 à 80% en poids de perséitol, plus avantageusement 25 à 70% en poids deperséitol.

De préférence, la composition relative en sucres de l'extrait hydrosoluble, en poids par rapport au poids total de la matière sèche de l'extrait, répond aux critères suivants (composition relative déterminée par HPLC) :

| | |
|---|---|
| - D-mannoheptulose | 25 à 60 % |
| - Perséitol | 25 à 60 % |
| - Saccharose | inférieur à 10 % |
| - Glucose | inférieur à 10 % |
| - Fructose | inférieur à 10 % |

L'extrait obtenu pourra éventuellement être lyophilisé dans le but d'obtenir une poudre solide (extrait sec), totalement hydrosoluble.

Selon une variante avantageuse de l'invention, le médicament selon l'invention comprend en outre un extrait peptidique de lupin, avantageusement en une quantité massique, par rapport au poids total du médicament, de 0,001 à 30% en poids sec, encore plus avantageusement de 0,01 à 10% en poids sec. L'extrait peptidique de lupin, ajouté dans la composition selon l'invention, comprend au moins 70%, avantageusement au moins 80%, en poids de peptides, par rapport au poids de la matière sèche de l'extrait peptidique. On observe alors avantagesement un effet de synergie.

En particulier, l'extrait peptidique de lupin est susceptible d'être obtenu par un procédé comprenant les étapes suivantes :
- préparation d'un tourteau de lupin broyé ou d'une farine de lupin micronisée ;
- puis, délipidation par extraction avec un solvant
- extraction des fractions protéiques et osidiques solubles, ou précipitation des protéines au point isoélectrique ;
- éventuellement séparation de la fraction protéique ;
   - hydrolyse enzymatique de la fraction protéique, et récupération, éventuellement après filtration, de l'extrait peptidique.

Le procédé de préparation d'un extrait peptidique est décrit dans le brevet FR 2 792 202, déposé par les laboratoires Expanscience.

Le médicament selon l'invention peut en outre comprendre au moins un composé choisi dans le groupe constitué par les émollients, les actifs hydratants, les activateurs de la synthèse de kératine, les kératorégulateurs, les kératolytiques, les agents restructurant de la barrière cutanée (activateurs de la synthèse des lipides cutanés, agonistes PPARs ou Peroxysome Proliferator Activated Receptor), les activateurs de la différenciation des kératinocytes (rétinoïdes, calcidone®, le calcium), les antibiotiques, les agents anti-bactériens, les composés antifongiques, les agents anti-viraux, les sébo-régulateurs, tels que les inhibiteurs de 5-alpha réductase, notamment l'actif 5-alpha Avocuta® commercialisé par les Laboratoires Expanscience, les immunomodulateurs, tels que le tacrolimus, le pimécrolimus, les oxazolines, les conservateurs, les agents anti-irritants, les agents apaisants, des filtres et écrans solaires, les agents anti-oxydants, les facteurs de croissance, les agents cicatrisants ou les molécules eutrophiques, les médicaments et les agents anti-inflammatoires, et les composés contenant des insaponifiables d'huiles végétales.

Les activateurs de la synthèse de kératine pouvant être utilisés dans le cadre de la présente invention en association avec l'extrait peptidique d'avocat sont avantageusement les rétinoïdes, les peptides de lupin (commercialisés par la société Silab), des protéines clés du stratum corneum ou granulosum (kératines).

Les antibiotiques pouvant être utilisés dans le cadre de la présente invention en association avec l'extrait peptidique d'avocat sont avantageusement l'acide fucidique, la pénicilline, les tétracyclines, la pristinamycine, l'érythromycine, la clindamycine, la mupirocine, la minocycline, la doxycycline. Les agents anti-viraux pouvant être utilisés dans le cadre de la présente invention en association avec l'extrait peptidique d'avocat sont avantageusement l'acyclovir et le valacyclovir. Les agents anti-irritants pouvant être utilisés dans le cadre de la présente invention en association avec l'extrait peptidique d'avocat sont avantageusement la glycine, les sucres et/ou peptides de lupin, le Cyclocéramide®.

Les agents apaisants pouvant être utilisés dans le cadre de la présente invention en association avec l'extrait peptidique d'avocat sont avantageusement l'alpha bisabolol, les dérivés de réglisse. Les kératorégulateurs pouvant être utilisés dans le cadre de la présente invention en association avec l'extrait peptidique d'avocat sont avantageusement les alpha hydroxy acides et leurs dérivés. Un kératolytique pouvant être utilisé dans le cadre de la présente invention en association avec l'extrait peptidique d'avocat est notamment l'acide salicylique et ses dérivés. Les agents anti-oxydants pouvant être utilisés dans le cadre de la présente invention en association avec l'extrait peptidique d'avocat sont avantageusement les vitamines (C, E), les oligo-éléments (cuivre, zinc, sélénium). Les facteurs de croissance pouvant être utilisés dans le cadre de la présente invention en association avec l'extrait peptidique d'avocat sont avantageusement la becaplermine et le TGF-beta (Transforming Growth Factor beta).

Les agents cicatrisants pouvant être utilisés dans le cadre de la présente invention en association avec l'extrait peptidique d'avocat sont avantageusement la vitamine A, le panthénol, l'Avocadofurane®, l'oxyde zinc, le magnésium, le silicium, l'acide madécassique ou asiatique.

Les médicaments pouvant être utilisés dans le cadre de la présente invention, en association avec l'extrait peptidique d'avocat, sont avantageusement les médicaments, appropriés pour une administration pour voie topique ou orale, pour la prévention et/ou le traitement de l'atopie (corticoïdes, émollients), de l'acné (antibiotiques, péroxyde de benzoyle, rétinoïdes, acide azélaïque, vitamine PP, zinc, cyclines), de l'eczéma (immunomodulateurs, émollients, huile de saumon, de bourrache, les pré-biotiques) ou du psoriasis (corticoïdes, calcipotriol, calcitriol, tazarotène, huile de cade, acitrétine, PUVA thérapie).

Les agents anti-inflammatoires pouvant être utilisés dans le cadre de la présente invention en association avec l'extrait peptidique d'avocat sont avantageusement des agents anti-inflammatoires stéroïdiens (AIS), tels que les corticoïdes, ou non-stéroïdiens (AINS). Les agents restructurant de la barrière cutanée, permettant de stimuler la synthèse des lipides clés de l'épiderme, et pouvant être utilisés dans le cadre de la présente invention en association, avantageusement avec un effet de synergie, avec l'extrait peptidique d'avocat sont avantageusement des concentrats de tournesol, encore plus avantageusement des concentrats de tournesol linoléiques, tels que l'actif commercialisé par les Laboratoires Expanscience, Soline® (cf. la demande internationale WO 01/21150), des insaponifiables d'huile végétale, tel que l'Avocadofurane® (cf. la demande internationale WO 01/21150), des agonistes PPARs (rosiglitazone, pioglitazone). Les agents restructurants sont avantageusement présent en une proportion allant de 0,001 à 30 % en poids, par rapport au poids total du médicament. Les composés antifongiques pouvant être utilisés dans le cadre de la présente invention en association avec l'extrait peptidique d'avocat sont avantageusement l'econazole et le ketoconazole.

Les conservateurs antiseptiques pouvant être utilisés dans le cadre de la présente invention en association avec l'extrait peptidique d'avocat sont par exemple le triclosan, la chlorhéxidine, les ammoniums quaternaires.

Les immunomodulateurs pouvant être utilisés dans le cadre de la présente invention en association avec l'extrait peptidique d'avocat sont avantageusement le tacrolimus, le pimécrolimus et les oxazolines.

Les oxazolines pouvant être utilisées dans le cadre de la présente invention en association, avantageusement avec un effet de synergie, avec l'extrait peptidique d'avocat sont avantageusement des oxazolines choisies dans le groupe constitué par la 2-undécyl-4-hydroxyméthyl-4-méthyl-1,3-oxazoline, la 2-undécyl-4,4-diméthyl-1,3-oxazoline, la (E)-4,4-diméthyl-2-heptadéc-8-ényl-1,3-oxazoline, la 4-hydroxyméthyl-4-méthyl-2-heptadécyl-1,3-oxazoline, la (E)-4-hydroxyméthyl-4-méthyl-2-heptadéc-8-ényl-1,3-oxazoline, la 2-undécyl-4-éthyl-4-hydroxyméthyl-1,3-oxazoline. De manière encore plus avantageuse, ladite oxazoline est la 2-undécyl-4,4-diméthyl-1,3-oxazoline, appelée OX-100 ou Cyclocéramide®.

Les composés contenant des insaponifiables d'huiles végétales pouvant être utilisés dans le cadre de la présente invention en association, avantageusement avec un effet de synergie, avec l'extrait peptidique d'avocat sont avantageusement choisis dans le groupe constitué par les lipides furaniques d'avocat, les insaponifiables d'avocat et de soja, les concentrats d'huile de lupin, les concentrats d'huile de tournesol et leurs mélanges.

Les lipides furaniques d'avocat pouvant être utilisés dans le cadre de la présente invention en association, avantageusement avec un effet de synergie, avec l'extrait peptidique d'avocat sont avantageusement des 2-alkyl furanes naturels, notamment l'actif Avocadofurane® commercialisé par les Laboratoires Expanscience, pouvant être obtenus par le procédé décrit dans la demande internationale WO 01/21605.

Les insaponifiables d'avocat et de soja pouvant être utilisés dans le cadre de la présente invention en association, avantageusement avec un effet de synergie, avec l'extrait peptidique d'avocat sont avantageusement un mélange d'insaponifiables d'avocat furanique et d'insaponifiables de soja, dans un rapport respectif d'environ 1/3-2/3. Les insaponifiables d'avocat et de soja sont encore plus avantageusement le produit Piasclédine®, commercialisé par les Laboratoires Expanscience.

Les concentrats d'huile de lupin pouvant être utilisés dans le cadre de la présente invention en association, avantageusement avec un effet de synergie, avec l'extrait peptidique d'avocat sont avantageusement des concentrats obtenus par distillation moléculaire d'huile de lupin, avantageusement d'huile de lupin blanc doux , tels que ceux décrits dans la demande internationale WO 98/47479. Ils contiennent avantageusement environ 60% en poids d'insaponifiables.

Les concentrats d'huile de tournesol pouvant être utilisés dans le cadre de la présente invention en association, avantageusement avec un effet de synergie, avec l'extrait peptidique d'avocat sont avantageusement des concentrats de tournesol linoléiques, tels que l'actif commercialisé par les Laboratoires Expanscience, Soline® (cf. la demande internationale WO 01/21150).

Le médicament selon l'invention est destiné au traitement et/ou à la prévention de maladies pouvant affecter l'être humain et/ou les animaux, notamment les mammifères. Le médicament selon l'invention peut être formulé sous la forme de différentes préparations adaptées à une administration topique, à une administration orale, rectale, vaginale, nasale, auriculaire ou bronchique, à une administration parentérale. De préférence les différentes préparations sont adaptées à l'administration topique et incluent les crèmes, les pommades, les lotions, les huiles, les patches, les sprays ou tout autres produits pour application externe. Les modes d'administration, les posologies et les formes galéniques optimales des composés et compositions selon l'invention peuvent être déterminés selon les critères généralement pris en compte dans l'établissement d'un traitement pharmaceutique, en particulier dermatologique ou vétérinaire, adapté à un patient ou à un animal comme par exemple l'âge ou le poids corporel du patient ou de l'animal, la gravité de son état général, la tolérance au traitement, les effets secondaires constatés, le type de peau. En fonction du type d'administration souhaitée, le médicament et/ou les composés actifs selon l'invention peuvent en outre comprendre au moins un excipient pharmaceutiquement acceptable, notamment dermatologiquement acceptable. De préférence, on utilise un excipient adapté pour une administration par voie topique externe. Le médicament selon la présente invention peut en outre comprendre au moins un adjuvant pharmaceutiquement connu de l'homme du métier, choisi parmi les épaississants, les conservateurs, les parfums, les colorants, des filtres chimiques ou minéraux, les agents hydratants, les eaux thermales, etc.

La présente invention concerne également une composition cosmétique comprenant un extrait peptidique d'avocat et un excipient approprié cosmétiquement acceptable. L'extrait peptidique d'avocat comprend 2 à 10% en poids d'azote alpha-aminé, par rapport au poids de la matière sèche de l'extrait peptidique.

La composition cosmétique selon l'invention comprend avantageusement 0,001 à 30 % en poids sec d'extrait peptidique d'avocat, par rapport au poids total de ladite composition, encore plus avantageusement 0,01% à 10 % en poids sec d'extrait peptidique d'avocat. Selon une variante avantageuse de l'invention, l'extrait peptidique d'avocat peut être obtenu selon un procédé tel que décrit précédemment.

Selon une variante avantageuse de l'invention, la composition comprend en outre du D-mannoheptulose et/ou du perséitol (effet de synergie), avantageusement en une quantité de 0,001 à 30 % en poids sec, encore plus avantageusement 0,01 à 5% en poids sec, par rapport au poids total de la composition. La source de D-mannoheptulose et/ou de perséitol est avantageusement un extrait hydrosoluble de sucres d'avocat, dont la teneur en sucres en C7, c'est-à-dire en D-mannoheptulose et en perséitol, dans la matière sèche est avantageusement comprise entre 65 à 90% en poids, par rapport au poids total de la matière sèche. Ces sucres de type heptitol peuvent aussi être obtenus à partir d'une autre source végétale ou par synthèse.

Selon une variante avantageuse de l'invention, la composition comprend en outre un extrait peptidique de lupin (effet de synergie), avantageusement en une quantité de 0,001 à 30 % en poids sec, par rapport au poids total de la composition. L'extrait peptidique de lupin, ajouté dans la composition selon l'invention, comprend au moins 70%, avantageusement au moins 80%, en poids de peptides, par rapport au poids de la matière sèche de l'extrait peptidique. Il peut être obtenu selon un procédé tel que décrit précédemment.

La composition peut en outre comprendre au moins un composé choisi dans le groupe constitué par les agents restructurant de la barrière cutanée et les composés contenant des insaponifiables d'huiles végétales, tels que définis précédemment. En particulier, la composition cosmétique peut comprendre un actif choisi dans le groupe constitué par Soline®, Avocadofurane® et Piasclédine®, commercialisées par les Laboratoires Expanscience.

La composition cosmétique selon l'invention comprend avantageusement 0,001 à 30 % en poids, par rapport au poids total de la composition, d'au moins un agent restructurant de la barrière cutanée.

La composition cosmétique selon l'invention peut être formulée sous la forme de différentes préparations adaptées à une administration topique, à une administration orale, rectale, ou vaginale, à une administration parentérale. De préférence les différentes préparations sont adaptées à l'administration topique et incluent les crèmes, les pommades, les lotions, les huiles, les patches, les sprays ou tout autres produits pour application externe. En fonction du type d'administration souhaitée, la composition et/ou les composés actifs selon l'invention peuvent en outre comprendre au moins un excipient cosmétiquement acceptable. La composition cosmétique selon la présente invention peut en outre comprendre au moins un adjuvant cosmétiquement connu de l'homme du métier, choisi parmi les épaississants, les conservateurs, les parfums, les colorants, des filtres chimiques ou minéraux, les agents hydratants, les eaux thermales, etc.

La présente invention concerne également une méthode de traitement cosmétique des peaux et/ou des muqueuses sensibles, irritées, intolérantes, présentant un trouble de la barrière cutanée, présentant des rougeurs cutanées ou présentant un déséquilibre immunologique non pathologique, caractérisée en ce qu'elle consiste à appliquer sur la peau et/ou les muqueuses une composition cosmétique selon l'invention.

La présente invention concerne également une méthode de traitement cosmétique des peaux telle que définie dans les revendications.

La présente invention concerne enfin une composition nutraceutique comprenant un extrait peptidique d'avocat et le cas échéant un excipient approprié acceptable. L'extrait peptidique d'avocat comprend avantageusement 2 à 10% en poids d'azote alpha-aminé, par rapport au poids de la matière sèche de l'extrait peptidique.

La composition nutraceutique selon l'invention comprend avantageusement 0,001 à 30 % en poids d'extrait peptidique d'avocat, par rapport au poids total de ladite composition, encore plus avantageusement 0,01 à 10 % en poids d'extrait peptidique d'avocat. Selon une variante avantageuse de l'invention, l'extrait peptidique d'avocat peut être obtenu selon un procédé tel que décrit précédemment.

Selon une variante avantageuse de l'invention, la composition nutraceutique comprend en outre du D-mannoheptulose et/ou du perséitol (effet de synergie), avantageusement en une quantité de 0,001 à 30 % en poids sec, par rapport au poids total de la composition. La source de D-mannoheptulose et/ou de perséitol est avantageusement un extrait hydrosoluble de sucres d'avocat, dont la teneur en sucres en C7, c'est-à-dire en D-mannoheptulose et en perséitol, dans la matière sèche est avantageusement comprise en 65 à 90% en poids, par rapport au poids total de la matière sèche.

Selon une variante avantageuse de l'invention, la composition nutraceutique comprend en outre un extrait peptidique de lupin (effet de synergie) avantageusement en une quantité de 0,001 à 30 % en poids sec, par rapport au poids total de la composition. L'extrait peptidique de lupin, ajouté dans la composition selon l'invention, comprend au moins 70%, avantageusement au moins 80%, en poids de peptides, par rapport au poids de la matière sèche de l'extrait peptidique. Il peut être obtenu selon un procédé tel que décrit précédemment.

Les exemples suivants permettent d'illustrer l'invention, ils ne sont pas limitatifs.

### Exemple 1 : préparation d'un extrait peptidique d'avocat

50 kg d'avocats frais, de la variété Hass, sont coupés en fines lamelles de 2 à 5 mm d'épaisseur, noyau compris, à l'aide d'un trancheur à disque. L'outil de séchage est une étuve thermo-régulée à courant d'air chaud. Les avocats tranchés sont répartis sur une épaisseur de 4 à 5 cm sur des clayettes étagées. La température de séchage est fixée à 80°C, sa durée est quant à elle de 48 heures. Une fois séchés, les fruits sont soumis à une pression à froid. Cette opération est réalisée sur une petite presse Komet® de laboratoire.

Les 4 kg de fruits délipidés (tourteau) sont alors broyés à froid puis extraits à reflux, en présence de 25 litres d'éthanol. La poudre épuisée en lipides est alors récupérée par filtration sur Büchner et séchée à l'étuve à 50°C, pendant 5 heures.

Le tourteau est alors lavé à l'eau déminéralisée (10 litres) puis séparé par centrifugation. La fraction solide est reprise dans une solution aqueuse, acidifiée par HCl (pH fixé à 5), puis mise en présence de 2 % de cellulase par rapport à la matière sèche. La durée d'hydrolyse est fixée à 6 heures.

Le mélange est ensuite centrifugé à 2000 g en présence d'adjuvant (2,5% p/v). Le culot récupéré subit alors une seconde hydrolyse à pH 8,0, en présence de 0,5 % de protéase, à une température de 55°C, pendant 2 heures. L'hydrolyse est régulée à pH constant par ajout continu de soude 2M. La protéase est enfin dénaturée par chauffage pendant 10 minutes, à 85°C.

Le mélange obtenu est centrifugé et le surnageant filtré par passage à travers une membrane de 7,5 µm. Il est ensuite ultrafiltré sur des membranes présentant un seuil de coupure de 10 kD.

L'extrait peptidique brut obtenu à 20 % de matière sèche, est décoloré en présence de 1% de charbon actif, puis filtré à nouveau à travers une membrane de 7,5 µm. L'extrait décoloré est alors microfiltré (0,2 µm), ajusté en titre à hauteur de 5 % de matière sèche, puis complémenté en conservateur et enfin conditionné après une filtration stérilisante (0,2 µm).

Les caractéristiques de l'extrait peptidique d'avocat hydrosoluble à 5 % de matière sèche, obtenu par ce procédé sont données dans le tableau 5 suivant :

**Tableau 5**

| Aspect | Solution de couleur légèrement orangée |
|---|---|
| **Critères analytiques** | |
| Matière sèche | 5% |
| pH (dilution ¼) | 4,5 |
| Absorbance à 420 nm (dilution ¼) | 0,152 |
| Absorbance à 550 nm (dilution ¼) | 0,035 |
| **Composition de la matière sèche** | |
| Azote alpha-aminé | 6,7 % |
| Protéines | Non détectées |
| Conservateur | 0,4 % |

Dans le tableau 6 suivant, on donne la répartition des masses moléculaires dans l'extrait peptidique d'avocat obtenu par ce procédé :

**Tableau 6**

| Pic HPLC | Masse molaire (g/mol) | Nombre d'acides aminés moyen | % relatif |
|---|---|---|---|
| 1 | > 3480 (1) | < 29 | 1 % |
| 2 | 3480 à 1180 | 29 à 9 | 26 % |
| 3 | 1180 à 310 | 9 à 2 | 45 % |
| 4 | 310 à 130 | 2 à 1 | 15 % |
| 5 | < 130 | 1 | 13 % |

| | | | |
|---|---|---|---|
| (1) masses < 10 000 g/mol. | | | |

On constate que 27 % au moins des peptides de l'extrait sont constitués d'au moins 9 motifs d'acides aminés. Par conséquent, la taille des peptides de l'extrait est donc extrêmement petite par comparaison à celle des protéines natives de l'avocat. Ces peptides présentent donc à ce titre une bien meilleure bio disponibilité, notamment cutanée.

### Exemple 2 : préparation d'un extrait hydrosoluble de sucres d'avocat

50 kg d'avocats frais, de la variété Hass, sont coupés en fines lamelles de 2 à 5 mm d'épaisseur, noyau compris, à l'aide d'un trancheur à disque. L'outil de séchage est une étuve thermo-régulée à courant d'air chaud. Les avocats tranchés sont répartis sur une épaisseur de 4 à 5 cm sur des clayettes étagées. La température de séchage est fixée à 80°C, sa durée est quant à elle de 48 heures. Une fois séchés, les fruits sont soumis à une pression à froid. Cette opération est réalisée sur une petite presse Komet® de laboratoire.

Les 4 kg de fruits délipidés (tourteau) sont alors broyés à froid puis extraits à reflux, en présence de 25 litres d'éthanol. La poudre épuisée en lipides est alors récupérée par filtration sur Büchner et séchée à l'étuve à 50°C, pendant 5 heures.

Le tourteau est alors lavé à l'eau déminéralisée (10 litres) puis séparé par centrifugation. La fraction soluble (liquide) est reprise pour être purifiée et concentrée selon le mode opératoire suivant :
- *Déminéralisation à l'aide de résines échangeuses d'ions :* déminéralisation des heptuloses par passage sur résines OH⁻, puis sur résine H⁺.
- *Ultrafiltration sur 10 000 Da :* l'ultrafiltration est réalisée avec un système équipé de 4 membranes de seuil de coupure 10 kDa.
- *Concentration sous vide :* la concentration de l'extrait purifié est réalisée à l'aide d'un évaporateur sous vide jusqu'à l'obtention d'une matière sèche voisine de 4 %.
- *Conditionnement:* la concentration de l'extrait est ajustée à 5 % de matière sèche et on ajoute du conservateur, puis on filtre stérilement avec une membrane de 0,2 µm de seuil de coupure et on conditionne.

Le tableau 7 suivant donne la composition de l'extrait de sucres d'avocat en C7, à 5 % de matière sèche, préparé suivant le procédé décrit ci-dessus :

**Tableau 7**

| **Aspect** | Solution de couleur jaune pâle |
|---|---|
| **Critères analytiques** | |
| Matière sèche | 5% |
| pH (dilution ¼) | 7,0 |
| Absorbance à 420 nm (dilution ¼) | 0,013 |
| Absorbance à 550 nm (dilution ¼) | 0,003 |
| **Composition (%/matière sèche)** | |
| Saccharose | 3,0 |
| Glucose | 7,5 |
| D-mannoheptulose | 40,0 |
| Fructose | 10,6 |
| Perséitol | 28,8 |

### Exemple 3 : Induction de la Bêta Défensine-2 par l'extrait peptidique d'avocat

### I. Ensemencement des cellules (J0) :

Des kératinocytes humains normaux sont ensemencés en plaque 96 puits (environ 20 000 cellules/puits), en présence d'un milieu spécifique enrichi en calcium (concentration finale 1,3 mM), tel que précédemment décrit dans la publication « Human β-Defensin-2 production in Keratinocytes is regulated by Interleukin-1, Bacteria, and the State of Differentiation », Alice Y. Liu et al., The Society for Investigative Dermatology, vol. 118, No. 2, Feb. 2002, pages 275 à 281.

### II. Traitement des cellules (J1) :

Après une incubation de 24h à 37°C, 5% CO₂ :
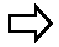 2 rinçages avec 200 µl/puits de PBS (tampon phosphate en solution saline) 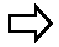
stimulation des cellules par 200 µl/puits (dans milieu supplémenté en Ca⁺⁺):
   ▪ par l'extrait peptidique d'avocat à des concentrations de 3, 1 et 0,3% soit respectivement 0,15, 0,05 et 0,015% de matière sèche
   ▪ par Il-1β à une concentration 100 ng/ml (contrôle positif d'induction de hBD-2)

### III. Fin du traitement (J2) : ELISA

Après 24h d'incubation, l'induction de l'hBD-2 est évaluée par une technique ELISA avec un anticorps spécifique (*goat polyclonal to human BD2 ; Abcam ; ab9871).*

Les résultats obtenus sont résumés dans le tableau 8 suivant :

**Tableau 8**

| | Cellules contrôle | Témoin positif (IL-1β) | Extrait peptidique d'avocat (0,3%) | Extrait peptidique d'avocat (1%) | Extrait peptidique d'avocat (3%) |
|---|---|---|---|---|---|
| hBD-2 (DO) | 0,03 | 0,103 | 0,057 | 0,047 | 0,05 |
| hBD-2 (DO) | 0,036 | 0,11 | 0,056 | 0,056 | 0,063 |
| hBD-2 (DO) | 0,036 | 0,105 | 0,062 | 0,054 | 0,072 |
| **Moyenne** | **0,034** | **0,106** | **0,058** | **0**,**052** | **0,062** |

On observe que, d'une manière tout à fait inattendue, l'extrait peptidique d'avocat selon l'invention permet d'augmenter la quantité de hBD-2 produite.

### Exemple 4 : induction des ARNm codant pour hBD-2 et pour les peptides anti-microbiens like (elastase specific inhibitor)

Nous avons utilisé la méthode des « cDNA micro array » pour étudier les effets de l'extrait peptidique d'avocat sur l'expression des gènes codant pour des protéines structurales et régulatrices d'intérêt potentiel dans la physiologie cutanée. Une telle approche permet de cribler en une seule étape les effets d'un produit ou d'un traitement sur l'expression des gènes dans un système biologique donné et d'avoir une signature des effets de ce traitement.

### Conditions de culture et produits à l'essai

Les extraits peptidiques d'avocat, à des concentrations de 3, 1 et 0,3% soit respectivement 0,15, 0,05 et 0,015% de matière sèche, obtenus par le procédé selon l'invention, ont été incubés directement dans le milieu de culture d'épidermes reconstruits Skinethic®, pendant 24h.

### Analyse de l'expression différentielle des gènes

La méthodologie utilisée, préconisée par Clontech (Palo Alto, USA), comprend :
▪ une étape d'extraction et de purification des ARN totaux
▪ une étape de purification des ARN messagers selon le protocole AtlasPure (Clontech)
▪ le marquage des sondes ADN au P³² par transcription inverse
▪ la purification des sondes marquées par chromatographie sur colonne d'exclusion et vérification de la qualité et de l'équivalence par comptage en scintillation liquide.
▪ l'hybridation des membranes (Custom ATLAS BIOAlternative) avec les sondes radio-marquées (68°C, une nuit).

### Résultats

Les effets sur la synthèse des ARNm codant pour les peptides anti-microbiens (hBD-2) et peptides anti-microbien like (Elastase specific inhibitor/SKALP/elafin) sont données dans le tableau 9 suivant :

**Tableau 9**

| **GENES** | Extrait peptidique d'avocat, actif pur (0,015%) | Extrait peptidique d'avocat, actif pur (0,05%) | Extrait peptidique d'avocat, actif pur (0,15%) |
|---|---|---|---|
| Beta défensine 2 | 16,1 | 69,7 | 103,9 |
| inhibiteur specifique de l'Elastase | 5,5 | 14,7 | 13,5 |

### Exemple 5 : formulations cosmétiques à base d'extrait peptidique d'avocat

| | Crème anti acné n°1 |
|---|---|
| Eau | QSP 100% |
| Isononyl Isononanoate | 7,000 |
| Di-C₁₂₋₁₃ Alkyl Malate | 7,000 |
| Stéarate isocétyle | 5,000 |
| Butylène Glycol | 3,000 |
| Oriza Sativa | 2,500 |
| **Extrait peptidique d'avocat** | **2,000** |
| Dicaprylyl Ether | 2,000 |
| Salicylate de silanediol | 2,000 |
| Alcool arachique | 1,650 |
| Tromethamine | 1,180 |
| Alcool cétylique | 1,000 |
| Acide salicylique | 1,000 |
| Glucoside Ascorbyl | 1,000 |
| Glycine | 1,000 |
| Acétate de Tocopheryl | 1,000 |
| Alcool béhénylique | 0,900 |
| Squalane | 0,790 |
| Citrate de sodium | 0,660 |
| Copolymère PPG-12/SMDI | 0,500 |
| Glucoside Arachidyl | 0,450 |
| Parfum | 0,400 |
| Gomme Sclerotium | 0,160 |
| Alcool cétéarylique | 0,130 |
| Acide citrique | 0,110 |
| Sepigel 305* | 0,100 |
| Système conservateur | QS |

| | |
|---|---|
| **produit commercialisé par le société Seppic* | |

| | Crème anti acné n°2 |
|---|---|
| Eau | QSP 100% |
| Isononyl Isononanoate | 7,000 |
| Di-C₁₂₋₁₃ Alkyl Malate | 7,000 |
| Stéarate isocétyle | 5,000 |
| Butylène Glycol | 3,000 |
| Oriza Sativa | 2,500 |
| **Extrait peptidique d'avocat** | **2,000** |
| **Sucres en C7 (heptitol)** | **1,000** |
| Dicaprylyl Ether | 2,000 |
| Salicylate de silanediol | 2,000 |
| Alcool arachique | 1,650 |
| Tromethamine | 1,180 |
| Alcool cétylique | 1,000 |
| Acide salicylique | 1,000 |
| Glucoside Ascorbyl | 1,000 |
| Glycine | 1,000 |
| Acétate de Tocopheryl | 1,000 |
| Alcool béhénylique | 0,900 |
| Squalane | 0,790 |
| Citrate de sodium | 0,660 |
| Copolymère PPG-12/SMDI | 0,500 |
| Glucoside Arachidyl | 0,450 |
| Parfum | 0,400 |
| Gomme Sclerotium | 0,160 |
| Alcool cétéarylique | 0,130 |
| Acide citrique | 0,110 |
| Sepigel 305* | 0,100 |
| Système conservateur | QS |

| | |
|---|---|
| **produit commercialisé par la société Seppic* | |

| | Emulsion moussante lavante pour peaux atopiques |
|---|---|
| Eau | QSP 100 |
| Arlatone duo* | 20,00000 |
| Glucoside de Coco | 12,00000 |
| Guar Hydroxypropyl | 2,00000 |
| **Extrait peptidique d'avocat** | **2,000** |
| Palmate de PEG-200 Glyceryl hydrogéné | 1,10000 |
| Cocoate de PEG-7 Glyceryl | 1,10000 |
| Salicylate de Silanediol | 1,00000 |
| Cocamide DEA | 1,00000 |
| Glycine de Caprylyol | 0,50000 |
| Sorbate de Potassium | 0,50000 |
| Polyquaternium 10 | 0,40000 |
| Parfum | 0,40000 |
| Acide citrique | 0,30000 |
| Zinc PCA | 0,20000 |

| | |
|---|---|
| **produit commercialisé par la société Quimasso* | |

| Emulsion moussante lavante pour hygiène intime | |
|---|---|
| Eau | QSP 100 |
| Arlatone duo* | 20,00000 |
| Glucoside de Coco | 12,00000 |
| Guar Hydroxypropyl | 2,00000 |
| **Extrait peptidique d'avocat** | **3,00000** |
| **Sucres d'avocat** | **1,00** |
| **Peptides de lupin** | **2,00** |
| Palmate de PEG-200 Glyceryl hydrogéné | 1,10000 |
| Cocoate de PEG-7 Glyceryl | 1,10000 |
| Salicylate de Silanediol | 1,00000 |
| Cocamide DEA | 1,00000 |
| Glycine de Caprylyol | 0,50000 |
| Sorbate de Potassium | 0,50000 |
| Polyquaternium 10 | 0,40000 |
| Parfum | 0,40000 |
| Acide citrique | 0,30000 |
| Zinc PCA | 0,20000 |

| | |
|---|---|
| **produit commercialisé par la société Quimasso* | |

## Revendications

1. Médicament comprenant un extrait peptidique d'avocat, qui comprend 2 à 10% en poids d'azote alpha-aminé par rapport au poids de la matière sèche de l'extrait peptidique, et un excipient approprié.

2. Médicament selon la revendication 1, **caractérisé en ce que** l'extrait peptidique d'avocat est susceptible d'être obtenu par un procédé comprenant les étapes suivantes :
- obtention d'un tourteau d'avocat, avantageusement du fruit de l'avocat, par séchage puis extraction de l'huile ; ensuite
- cryobroyage et délipidation complète dudit tourteau puis décantation, centrifugation et récupéreration du gâteau ; puis
- première hydrolyse en présence de glucanases, suivie d'une centrifugation et de l'élimination de la fraction soluble ;
- seconde hydrolyse en présence d'une ou plusieurs protéases, suivie d'une centrifugation et de l'élimination du culot ; ensuite
- concentration de la phase peptidique par nanofiltration et le cas échéant, décoloration en présence de charbon actif ; suivie
- d'une filtration simple (10 µm) puis d'une ultrafiltration (seuil de coupure de 10 kD) ; enfin
- le cas échéant, ajout de conservateur, microfiltration stérilisante finale (0,2 µm) et conditionnement.

3. Médicament selon la revendication 1 ou 2, **caractérisé en ce que** l'extrait peptidique d'avocat présente la composition suivante en acides aminés, en pourcentage en poids par rapport au poids total d'acides aminés :
| | |
|---|---|
| Alanine | 6,4 - 7,8 |
| Arginine | 4,7 - 5,7 |
| Acide aspartique | 10,3 - 12,7 |
| Cystine-cystéine | 2,9 - 3,5 |
| Acide glutamique | 13,0 - 15,8 |
| Glycine | 5,3 - 6,5 |
| Histidine | 2,2 - 2,6 |
| Isoleucine | 4,8 - 5,8 |
| Leucine | 7,6 - 9,4 |
| Lysine | 3,0 - 3,8 |
| Méthionine | 1,2 - 1,6 |
| Phénylalanine | 4,7 - 5,7 |
| Proline | 4,1 - 5,2 |
| Sérine | 5,5 - 6,7 |
| Thréonine | 4,6 - 5,6 |
| Tyrosine | 3,6 - 4,4 |
| Valine | 5,8 - 7,2 |

4. Médicament selon l'une quelconque des revendications précédentes, **caractérisé en ce que** 50% des peptides de l'extrait sont constitués de 10 à 30 acides aminés.

5. Médicament selon l'une quelconque des revendications précédentes, pour son utilisation dans le traitement et/ou à la prévention de maladies liées à une modification de l'immunité innée, par augmentation de la production de peptides antimicrobiens, de la famille des cathélicidines et/ou des bétâ-défensine, avantageusement de la hBD-2.

6. Médicament selon l'une quelconque des revendications 1-4 pour son utilisation dans le traitement et/ou à la prévention de maladies liées à une modification de l'immunité innée par stimulation des peptides anti-microbiens like, tels qu'un inhibiteur spécifique de l'élastase, particulièrement l'élafin (SKALP).

7. Médicament pour une utilisation selon la revendication 5 ou 6, **caractérisé en ce que** lesdites maladies sont liées à la présence de micro-organismes tels que les bactéries Gram+ et/ou Gram-, les champignons ou les virus.

8. Médicament pour une utilisation selon l'une quelconque des revendications 5 à 7, **caractérisé en ce que** lesdites maladies sont choisies dans le groupe constitué par :
- des infections de la peau et des phanères, notamment choisies dans le groupe constitué par que la folliculite, le furoncle, l'abcès, l'impétigo ou le panaris ;
- des dermatoses inflammatoires, telles que la dermatite atopique, l'eczéma de contact et/ou atopique, le psoriasis, l'acné, les dermites irritatives ;
- des allergies des peaux et/ou des muqueuses ;
ou
- les brûlures.

9. Médicament pour une utilisation selon la revendication 5 ou 6, **caractérisé en ce que** lesdites maladies sont des pathologies liées à un déficit de la barrière cutanée, telles que les peaux hyper-réactives, atopiques, ou des pathologies liées à des peaux fragilisées par une agression environnementale.

10. Médicament selon l'une quelconque des revendications 1 à 4, ou pour une utilisation selon la revendications 5 ou 6, pour son utilisation dans la protection des peaux :
- pathologiques immatures des nourrissons ou des enfants ; ou
- pathologiques des individus adultes ou âgés.

11. Médicament selon l'une quelconque des revendications 1 à 4 ou pour une utilisation selon la revendication 5 ou 6, pour son utilisation pour favoriser la cicatrisation, dans les processus de cicatrisation normaux ou pathologiques, tels que les ulcères et les escarres.

12. Médicament selon l'une quelconque des revendications 1 à 4, ou pour une utilisation selon la revendication 5 ou 6, pour son utilisation dans le traitement et/ou la prévention des maladies parodontales.

13. Médicament selon l'une quelconque des revendications 1 à 4, ou pour une utilisation selon la revendication 5 ou 6, pour son utilisation dans le traitement et/ou la prévention des pathologies articulaires inflammatoires telle que l'arthrose.

14. Médicament selon l'une quelconque des revendications 1 à 4, ou pour une utilisation selon les revendication 5 ou 6, pour son utilisation dans le traitement et/ou la prévention des infections des muqueuses, notamment des muqueuses vaginale, intestinale, respiratoire, nasale ou auriculaire.

15. Médicament selon l'une quelconque des revendications 1 à 4 ou pour une utilisation selon la revendication 5 ou 6, pour son utilisation dans le traitement et/ou la prévention des infections du système oculaire.

16. Médicament selon l'une quelconque des revendications 1-4, ou pour une utilisation selon la revendication 5 ou 6, comprenant
en outre du D-mannoheptulose et/ou du perséitol, avantageusement la source de D-mannoheptulose et/ou de perséitol est un extrait hydrosoluble de sucres d'avocat.

17. Composition cosmétique ou nutraceutique comprenant un extrait peptidique d'avocat, qui comprend avantageusement 2 à 10% en poids d'azote alpha-aminé, par rapport au poids de la matière sèche de l'extrait peptidique, et un excipient approprié réciproquement cosmétiquement acceptable ou alimentaire.

18. Composition cosmétique ou nutraceutique selon la revendication 17, **caractérisée en ce qu'**elle comprend en outre du D-mannoheptulose et/ou du perséitol, avantageusement la source de D-mannoheptulose et/ou de perséitol est un extrait hydrosoluble de sucres d'avocat.

19. Méthode de traitement cosmétique des peaux et/ou des muqueuses sensibles, irritées, sèches, âgées, intolérantes, présentant un trouble de la barrière cutanée, fragilisées par une agression environnementale présentant des rougeurs cutanées ou présentant un déséquilibre immunologique non pathologique, **caractérisée en ce qu'**elle consiste à appliquer sur la peau et/ou les muqueuses une composition cosmétique selon l'une quelconque des revendications 17 et 18.

20. Méthode de traitement cosmétique des peaux saines immatures des nourrissons ou des enfants ou des peaux saines des individus adultes ou âgés, **caractérisée en ce qu'**elle consiste à appliquer sur la peau une composition cosmétique selon l'une quelconque des revendications 17 et 18.

## Patentansprüche

1. Arzneimittel, umfassend einen Avocado-Peptidextrakt, der 2 bis 10 Gew.-% inklusive Alpha-Aminstickstoff in Bezug auf das Gewicht der Trockenmasse des Peptidextrakts umfasst, und einen geeigneten Hilfsstoff.

2. Arzneimittel nach Anspruch 1, **dadurch gekennzeichnet, dass** der Avocado-Peptidextrakt anhand eines Verfahrens gewinnbar ist, das die folgenden Schritte umfasst:
- Gewinnen eines Avocadokuchens, in vorteilhafter Weise aus der Avocadofrucht, durch Trocknen und danach Extraktion des Öls; anschließend
- kryogenes Zerkleinern und Entfetten des Kuchens, dann Dekantieren, Zentrifugieren und Rückgewinnen des Kuchens; dann
- erste Hydrolyse in Anwesenheit von Glucanasen, gefolgt von einer Zentrifugierung und der Entfernung der löslichen Fraktion;
- zweite Hydrolyse in Anwesenheit von einer oder mehreren Proteasen, gefolgt von einer Zentrifugierung und der Entfernung des Rückstands; danach
- Konzentrieren der Peptidphase durch Nanofiltration und gegebenenfalls Entfärben in Anwesenheit von Aktivkohle; gefolgt
- von einer einfachen Filtration (10 µm), dann von einer Ultrafiltration (Cut-off-Wert 10 kD); schließlich
- gegebenenfalls Hinzufügen eines Konservierungsstoffs, abschließende sterilisierende Mikrofiltration (0,2 µm) und Konditionieren.

3. Arzneimittel nach Anspruch 1 oder 2, **dadurch gekennzeichnet, dass** der Avocado-Peptidextrakt die folgende Aminosäuren-Zusammensetzung in Gewichtsprozent in Bezug auf das Aminosäuren-Gesamtgewicht aufweist:
| | |
|---|---|
| Alanin | 6,4-7,8 |
| Arginin | 4,7-5,7 |
| Asparaginsäure | 10,3-12,7 |
| Cystin-Cystein | 2,9-3,5 |
| Glutaminsäure | 13,0-15,8 |
| Glycin | 5,3-6,5 |
| Histidin | 2,2-2,6 |
| Isoleucin | 4,8-5,8 |
| Leucin | 7,6-9,4 |
| Lysin | 3,0-3,8 |
| Methionin | 1,2-1,6 |
| Phenylalanin | 4,7-5,7 |
| Prolin | 4,1-5,2 |
| Serin | 5,5-6,7 |
| Threonin | 4,6-5,6 |
| Tyrosin | 3,6-4,4 |
| Valin | 5,8-7,2 |

4. Arzneimittel nach einem der vorangehenden Ansprüche, **dadurch gekennzeichnet, dass** 50 % der Peptide des Extrakts von 10 bis 30 Aminosäuren gebildet sind.

5. Arzneimittel nach einem der vorangehenden Ansprüche für seine Anwendung bei der Behandlung und/oder der Vorbeugung von Krankheiten, die mit einer Veränderung der angeborenen Immunität verbunden sind, durch Erhöhung der Produktion von antimikrobischen Peptiden aus der Familie der Cathelicidine und/oder der Beta-Defensine, in vorteilhafter Weise von hBD-2.

6. Arzneimittel nach einem der Ansprüche 1-4 für seine Anwendung bei der Behandlung und/oder der Vorbeugung von Krankheiten, die mit einer Veränderung der angeborenen Immunität verbunden sind, durch Stimulation der antimikrobischen-like Peptide, wie ein spezieller Inhibitor der Elastase, insbesondere Elafin (SKALP).

7. Arzneimittel für eine Verwendung nach Anspruch 5 oder 6, **dadurch gekennzeichnet, dass** die Krankheiten mit der Anwesenheit von Mikroorganismen wie die Gram+ und/oder Gram- Bakterien, die Pilze oder die Viren verbunden sind.

8. Arzneimittel für eine Verwendung nach einem der Ansprüche 5 bis 7, **dadurch gekennzeichnet, dass** die Krankheiten aus der Gruppe ausgewählt sind, die gebildet ist von:
- Hautinfektionen und Hautanhanggebilden, insbesondere ausgewählt aus der Gruppe, die von der Folliculitis, dem Furunkel, dem Abszess, der Impetigo oder der Nagelbettentzündung gebildet ist;
- entzündlichen Dermatosen wie die atopische Dermatitis, das Kontakt- und/oder atopische Ekzem, die Schuppenflechte, die Akne, die irritativen Dermatitiden;
- Allergien der Haut und/oder der Schleimhäute; oder
- den Verbrennungen.

9. Arzneimittel für eine Verwendung nach Anspruch 5 oder 6, **dadurch gekennzeichnet, dass** die Krankheiten Pathologien sind, die mit einem Defizit der Hautbarriere verbunden sind, wie die hyperreaktive, atopische Haut, oder Krankheiten, die mit durch Umweltschadstoffe geschwächter Haut verbunden sind.

10. Arzneimittel nach einem der Ansprüche 1 bis 4 oder für eine Verwendung nach Anspruch 5 oder 6 für seine Verwendung im Hautschutz:
- Unreifepathologien der Säuglinge oder der Kinder; oder
- Pathologien Erwachsener oder alter Menschen.

11. Arzneimittel nach einem der Ansprüche 1 bis 4 oder für eine Verwendung nach Anspruch 5 oder 6 für seine Verwendung zur Förderung der Wundheilung in normalen oder pathologischen Wundheilungsprozessen, wie die Geschwüre oder die Dekubitus.

12. Arzneimittel nach einem der Ansprüche 1 bis 4 oder für eine Verwendung nach Anspruch 5 oder 6 für seine Verwendung bei der Behandlung und/oder der Vorbeugung von Parodontalerkrankungen.

13. Arzneimittel nach einem der Ansprüche 1 bis 4 oder für eine Verwendung nach Anspruch 5 oder 6 für seine Verwendung bei der Behandlung und/oder der Vorbeugung von entzündlichen Gelenkerkrankungen wie die Arthrose.

14. Arzneimittel nach einem der Ansprüche 1 bis 4 oder für eine Verwendung nach Anspruch 5 oder 6 für seine Verwendung bei der Behandlung und/oder der Vorbeugung der Infektionen der Schleimhäute, vor allem der Schleimhäute der Vagina, des Darms, der Atmung, der Nase oder des Gehörs.

15. Arzneimittel nach einem der Ansprüche 1 bis 4 oder für eine Verwendung nach Anspruch 5 oder 6 für seine Verwendung bei der Behandlung und/oder der Vorbeugung von Infektionen des optischen Systems.

16. Arzneimittel nach einem der Ansprüche 1-4 oder für eine Verwendung nach Anspruch 5 oder 6, umfassend ferner D-Mannoheptulose und/oder Perseitol, wobei in vorteilhafter Weise die Quelle von D-Mannoheptulose und/oder von Perseitol ein wasserlöslicher Extrakt von Avocadozuckern ist.

17. Kosmetische oder nutrazeutische Zusammensetzung, umfassend einen Avocado-Peptidextrakt, der 2 bis 10 Gew.-% inklusive Alpha-Aminstickstoff in Bezug auf das Gewicht der Trockenmasse des Peptidextrakts umfasst, und einen geeigneten Hilfsstoff, der sowohl kosmetisch als auch in der Ernährung akzeptabel ist.

18. Kosmetische oder nutrazeutische Zusammensetzung nach Anspruch 17, **dadurch gekennzeichnet, dass** sie ferner D-Mannoheptulose und/oder Perseitol umfasst, wobei in vorteilhafter Weise die Quelle von D-Mannoheptulose und/oder von Perseitol ein wasserlöslicher Extrakt von Avocadozuckern ist.

19. Kosmetische Behandlungsmethode sensibler, gereizter, trockener, alter, intoleranter Haut und/oder Schleimhäute, die eine Störung der Hautbarriere aufweisen, durch Umweltschadstoffe geschwächt sind, die Hautrötungen oder ein nicht pathologisches immunologisches Ungleichgewicht aufweisen, **dadurch gekennzeichnet, dass** sie darin besteht, auf die Haut und/oder die Schleimhäute eine kosmetische Zusammensetzung nach einem der Ansprüche 17 und 18 aufzutragen.

20. Kosmetische Behandlungsmethode der unreifen gesunden Haut von Säuglingen oder Kindern oder der gesunden Haut von Erwachsenen oder alten Menschen, **dadurch gekennzeichnet, dass** sie darin besteht, auf die Haut eine kosmetische Zusammensetzung nach einem der Ansprüche 17 und 18 aufzutragen.

## Claims

1. A drug comprising an avocado peptide extract, which comprises 2-10 weight % alpha-aminated nitrogen, in relation to the peptide extract dry matter weight, and an appropriate excipient.

2. The drug according to claim 1, **characterized in that** the avocado peptide extract may be obtained by a process comprising the following steps:
- obtaining an avocado cake, advantageously from the avocado fruit, by drying and oil extraction; followed by
- cryogrinding and total delipidation of said cake, followed by decantation, centrifugation and collection of the cake; followed by
- a first hydrolysis in the presence of glucanases, followed by a centrifugation and discarding of the soluble fraction;
- a second hydrolysis in the presence of one or several proteases, followed by a centrifugation and discarding of the residue; followed by
- a concentration of the peptide phase by nanofiltration and, if need be, a discoloration in the presence of activated carbon; followed by
- a simple filtration (10 µm), followed by an ultrafiltration (cutoff limit 10 kD); and finally
- if need be, addition of a preservative, a final sterilizing microfiltration (0.2 µm) and packaging.

3. The drug according to claims 1 or 2, **characterized in that** the avocado peptide extract has the following amino acid composition, in weight percentage in relation to the total amino acid weight:
| | |
|---|---|
| Alanine | 6.4-7.8 |
| Arginine | 4.7-5.7 |
| Aspartic acid | 10.3-12.7 |
| Cystine-Cysteine | 2.9-3.5 |
| Glutamic acid | 13.0-15.8 |
| Glycine | 5.3-6.5 |
| Histidine | 2.2-2.6 |
| Isoleucine | 4.8-5.8 |
| Leucine | 7.6-9.4 |
| Lysine | 3.0-3.8 |
| Methionine | 1.2-1.6 |
| Phenylalanine | 4.7-5.7 |
| Proline | 4.1-5.2 |
| Serine | 5.5-6.7 |
| Threonine | 4.6-5.6 |
| Tyrosine | 3.6-4.4 |
| Valine | 5.8-7.2 |

4. The drug according to any of the preceding claims, **characterized in that** 50% of peptides in the extract are made up of 10-30 amino acids.

5. The drug according to any of the preceding claims, for its use in the treatment and/or the prevention of diseases which are linked to a change in natural immunity, by increasing the production of antimicrobial peptides, from the cathelicidins and/or beta-defensins families, advantageously hBD-2.

6. The drug according to any of claims 1-4, for its use in the treatment and/or the prevention of diseases which are linked to a change in natural immunity, by stimulating antimicrobial like peptides, such as a specific elastase inhibitor, particularly elafin (SKALP)

7. The drug for use according to claim 5 or 6, **characterized in that** said diseases are linked to the presence of microorganisms such as Gram+ and/or Gram-bacteria, fungi or viruses.

8. The drug for use according to any of claims 5-7, **characterized in that** said diseases are chosen among the group constituted by:
- infections of the skin and phanerae, notably chosen within the group which is made up by folliculitis, furuncles, abscess, impetigo or whitlow;
- inflammatory dermatoses, such as atopic derrmatitis, contact and/or atopic eczema, psoriasis, acne, itching dermatites;
- skins and/or mucosae's allergies; or
- burns.

9. The drug for use according to claim 5 or 6, **characterized in that** said diseases are pathologies which are linked to a deficiency in the skin barrier, such as hyperreactive or atopic skins, or pathologies linked to skins which have been weakened by an aggression from the environment.

10. The drug according to any of claims 1-4, or for use according to claim 5 or 6, for its use in the protection of skins such as:
- pathological immature newborn infants' or children's skins;
- pathological skins in adults or aged individuals.

11. The drug according to any of claims 1-4, or for use according to claim 5 or 6, for its use in enhancing the healing process in normal or pathological healing processes, such as ulcers and bedsores.

12. The drug according to any of claims 1-4, or for use according to claim 5 or 6, for its use in the treatment and/or the prevention of periodontal diseases.

13. The drug according to any of claims 1-4, or for use according to claim 5 or 6, for its use in the treatment and/or the prevention of inflammatory articular pathologies such as arthrosis.

14. The drug according to any of claims 1-4, or for use according to claim 5 or 6, for its use in the treatment and/or the prevention of infections of the mucosae, notably the vaginal, intestinal, respiratory, nasal or auricular mucosae.

15. The drug according to any of claims 1-4, or for use according to claim 5 or 6, for its use in the treatment and/or the prevention of the infections of the sight organs.

16. The drug according to any of the claims 1-4, or for use according to claim 5 or 6, further comprising D-mannoheptulose and/or perseitol, advantageously the source of D-mannoheptulose and/or perseitol being a hydrosoluble avocado sugar extract.

17. A cosmetic or nutraceutic composition comprising an avocado peptide extract, which advantageously contains 2-10 weight % alpha-aminated nitrogen, in relation to the peptide extract dry matter weight, and an appropriate excipient, which is, respectively cosmetically suitable or a food grade excipient.

18. The cosmetic or nutraceutic composition according to claim 17, **characterized in that** it further contains D-mannoheptulose and/or perseitol, advantageously the source of D-mannoheptulose and/or perseitol being an avocado sugar hydrosoluble extract.

19. A process for the cosmetic treatment of sensitive, irritated, dry, aged, intolerant skins and/or mucosae, with a deficiency in the skin barrier, which have been weakened by an aggression from the environment, with reddenings or with an non pathological immunological imbalance, **characterized in that** it consists in applying onto the skin and/or the mucosae a cosmetic composition according to any of claims 17-18.

20. The process for the cosmetic treatment of healthy immature newborn infants' or children's skins or healthy skins in adults or aged individuals, **characterized in that** it consists in applying onto the skin a cosmetic composition according to any of claims 17-18.
